# EUROPEAN PATENT SPECIFICATION

(11) **EP 2 109 419 B1**
(45) Date of publication and mention of the grant of the patent: **04.01.2017**
(21) Application number: 08729469.0
(22) Date of filing: 08.02.2008
(51) Int. Cl.: A61F 2/24

(54) **PROGRESSIVELY SIZED ANNULOPLASTY RINGS**
ANNULOPLASTIERINGE IN AUFSTEIGENDEN GRÖSSEN
ANNEAUX POUR ANNULOPLASTIE TAILLÉS PROGRESSIVEMENT

(30) Priority: 09.02.2007 US 889178 P
(43) Date of publication of application: 21.10.2009
(73) Proprietor: Edwards Lifesciences Corporation, Irvine, CA 92614 (US)
(72) Inventor: CARPENTIER, Alain, F-75116 Paris (FR); ADAMS, David, New York, NY 10029 (US)
(74) Representative: Eisenführ Speiser
(86) International application number: PCT/US2008/053513
(87) International publication number: WO 2008/098226

(56) References cited:
- WO-A-99/65423
- WO-A-2005/034813
- WO-A-2005/110290

## Description

### Field of the Invention

The present application claims priority under 35 U.S.C. §119(e) to U.S. Provisional Application No. 60/828,458, filed October 6, 2006.

The present invention refers to a prosthetic annuloplasty ring or set of rings, in particular for the mitral annulus, that are progressively proportioned at different orifice sizes.

### Background of the Invention

The human heart has four valves; the aortic valve, the mitral valve, the pulmonary valve and the tricuspid valve. Various diseases and certain genetic defects of the heart valves can impair the proper functioning of the valves. Improper functioning of a valve can be severely debilitating and even fatal if left untreated, particularly if the diseased valve is the aortic valve (between the left ventricle and the aorta) or the mitral valve (between the left atrium and left ventricle). The common defects and diseases affecting each of these valves, and the treatments thereof, are typically different.

The mitral valve and, less frequently, the tricuspid valve, are prone to deformation, such as dilation of the valve annulus, tearing of the chordae tendineae and leaflet prolapse, which results in valvular insufficiency wherein the valve does not close properly and allows for regurgitation or back flow from the left ventricle into the left atrium. Deformations in the structure or shape of the mitral or tricuspid valve are repairable. Thus, because prosthetic valves have certain disadvantages that can have serious effects (e.g., mechanical valves carry the risk of thromboembolism and require anticoagulation treatment, and biological valves have limited durability), an improper functioning mitral or tricuspid valve is ideally repaired rather than replaced.

A remodelling mitral annuloplasty ring having a generally oval shaped body with a major axis perpendicular to a minor axis, both perpendicular to a blood flow axis is disclosed in WO2005/034813. The ring is provided in sets of different orifice sizes.

The mitral annulus is a pliable junctional zone of fibrous and muscular tissue joining the left atrium and left ventricle that anchors the peripheral hinge portion of the anterior and posterior mitral leaflets. The annulus has two major collagenous structures: (1) the right fibrous trigone, which is part of the central fibrous body and is located at the intersection of the atrioventricular membranous septum, the mitral and tricuspid valves, and the aortic root; and (2) the left fibrous trigone at the junction of the mitral valve and left coronary cusp of the aortic valve. The mitral valve has two major leaflets, the much larger anterior (or aortic) leaflet and the smaller posterior (or mural) leaflet. The anterior mitral leaflet spans the distance between the commissures (including the trigones) and is in direct fibrous continuity with most of the left and noncoronary aortic valve cusps. The posterior one-half to two-thirds of the annulus, which subtends the posterior leaflet, is primarily muscular with little or no fibrous tissue, and usually contains three (or sometimes more) scallops separated by fetal clefts or "subcommissures."

During systolic contraction of the heart, the free margins of the mitral leaflets appose each other and close the respective atrial-ventricular passage. The chordae tendineae and papillary muscles hold the leaflets in this position throughout the systole cycle to prevent the leaflets from bulging into and opening within the left atrium. The functional competence of the mitral valve relies on proper, coordinated interaction of the mitral annulus and leaflets, chordae tendineae, papillary muscles, left atrium, and left ventricle. However, when the valve or its leaflets are misshapen or enlarged, for example, when the annulus is dilated, the edges of the leaflets fail to meet each other, leaving an opening therebetween. This opening may involve lateral separation of the valve leaflets and/or elevation of one valve leaflet with respect to the other. In either case, the ineffective closure of the valve during ventricular contraction results in regurgitation or leakage of blood back into the atrium, and ultimately in reduced pumping efficiency. To compensate for such inefficiency in the mitral valve, the left ventricle must work harder to maintain the requisite cardiac output. Over time, this compensatory mechanism typically results in hypertrophy of the heart followed by dilation, i.e., an enlarged heart, which can lead to congestive heart failure.

Mitral regurgitation is one of the most common valvular malfunctions in the adult population, and typically involves the elongation or dilation of the posterior two-thirds of the mitral valve annulus, the section corresponding to the posterior leaflet. The most common etiology of systolic mitral regurgitation in patients undergoing surgical evaluation is myxomatous degeneration, also termed mitral valve prolapse (29% to 70% of cases), or in gross terms, at least 5 to 10 percent of the population in the U.S. Women are affected about twice as often as men. Mitral valve prolapse has been diagnosed as Barlow's syndrome, billowing or balloon mitral valve, floppy mitral valve, floppy-valve syndrome, myxomatous mitral valve, prolapsing mitral leaflet syndrome, or systolic click-murmur syndrome. The syndrome of mitral valve prolapse includes palpitations, chest pain, syncope or dyspnea, and a mid-systolic click (with or without a late systolic murmur of mitral regurgitation). These latter findings are typically seen in patients with Barlow's syndrome, where extensive hooding and billowing of both leaflets are the rule. Some forms of mitral valve prolapse seem to be hereditary, though the condition has been associated with Marfan's syndrome, Grave's disease, and other disorders.

Myxomatous degeneration involves weakness in the leaflet structure, leading to thinning of the tissue and loss of coaptation. Barlow's disease is characterized by myxoid degeneration and appears early in life, often before the age of fifty. In Barlow's disease, one or both leaflets of the mitral valve protrude into the left atrium during the systolic phase of ventricular contraction. The valve leaflets are thick with considerable excess tissue, producing an undulating pattern at the free edges of the leaflets. The chordae are thickened, elongated and may be ruptured. Papillary muscles are also occasionally elongated. The annulus is dilated and sometimes calcified. Of course, some of these symptoms present in other pathologies, and therefore the present application will refer to mitral valve prolapse as a catch-all for the various diagnoses, including Barlow's syndrome.

Other causes of mitral regurgitation include ischemic heart disease with ischemic mitral regurgitation (IMR), dilated cardiomyopathy (in which the term "functional mitral regurgitation" [FMR] is used), rheumatic valve disease, mitral annular calcification, infective endocarditis, idiopathic chordal rupture (usually associated with fibroelastic deficiency [FED]), congenital anomalies, endocardial fibrosis, and collagen-vascular disorders. IMR is a specific subset of FMR, but both are usually associated with morphologically normal mitral leaflets.

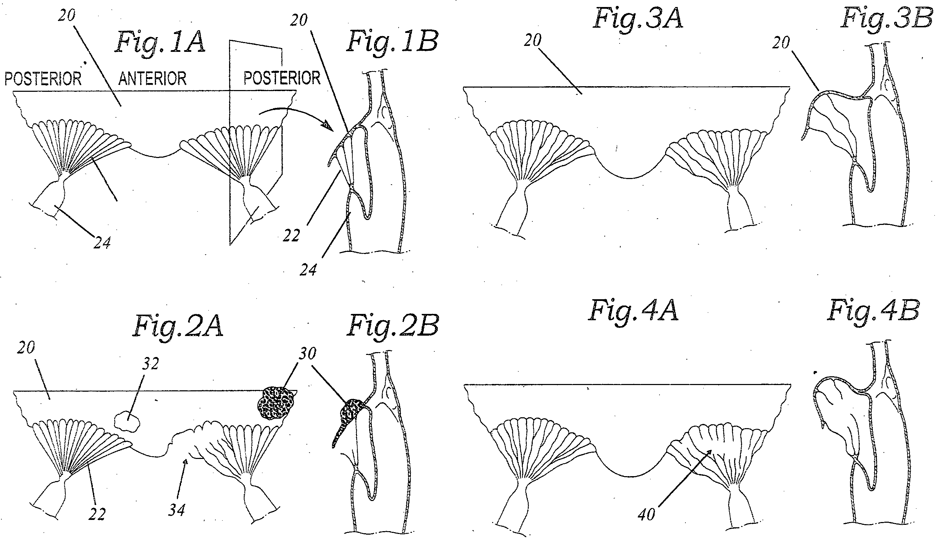

It will therefore be apparent that the types of valve disease that lead to regurgitation are varied and present vastly differently. For instance, Figures 1-8 show first a normal mitral valve anatomy and then the causes of pure mitral regurgitation from a number of pathologies. Figures 1A-1B show a normal mitral anatomy with the mitral leaflet 20 spread out plat in Figure 1A, and Figure 1B shown as a section through one papillary muscle 22. The chordae 24 connect the lower edges of the leaflet 20 to the papillary muscles 22 within the left ventricle.

Figures 2A-2B illustrate a condition diagnosed as infective endocarditis, either active or healed. Vegetation or growths 30 may occur on the leaflet 20, and sometimes a perforation 32. Often the chordae 24 rupture, such as at 34.

Figures 3A-3B illustrate floppy mitral valve which causes prolapse. The leaflets 20 is distended, increasing the annulus area, leaflet area, and causing buckling. Figures 4A-4B show advanced floppy mitral valve which causes the chordae to rupture, such as seen at 40.

Figures 5A-5B illustrate rheumatic heart disease. Diffuse fibrous thickening forms at the lower edge of the mitral leaflet 20 and the chordae 24 exhibit focal thickening.

Figures 6A-6B illustrate papillary muscle dysfunction (coronary), in which one or more of the muscles is scarred and atrophied, such as at 50. Possible effects may be severe coronary artery narrowing and acute or healed infarct.

Figures 7A-7B illustrate papillary muscle dysfunction (infiltrative), in which typically both muscles are infiltrated with foreign bodies, possibly amyloid, sarcoid, infection or neoplasm.

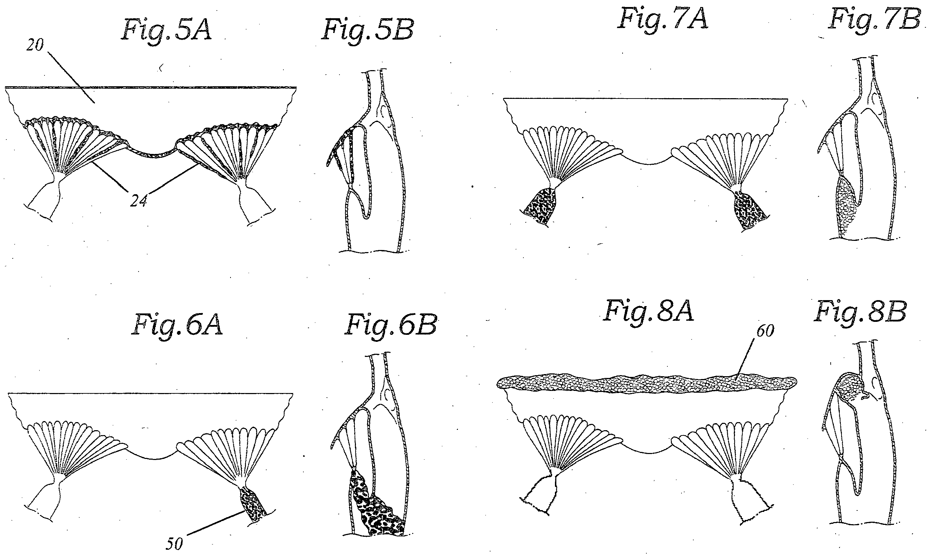

Finally, Figures 8A-8B annular calcification. Calcific deposits 60 produce leaflet protrusion toward the atrium.

As is clear from the illustrations 2-8, many conditions lead to regurgitation. However, it is understood that four general types of structural changes of the mitral valve apparatus may produce regurgitation: leaflet retraction from fibrosis and calcification, annular dilation, chordal abnormalities (including rupture, elongation, shortening, or apical tethering or "tenting" as seen in FMR and IMR), and possibly papillary muscle dysfunction.

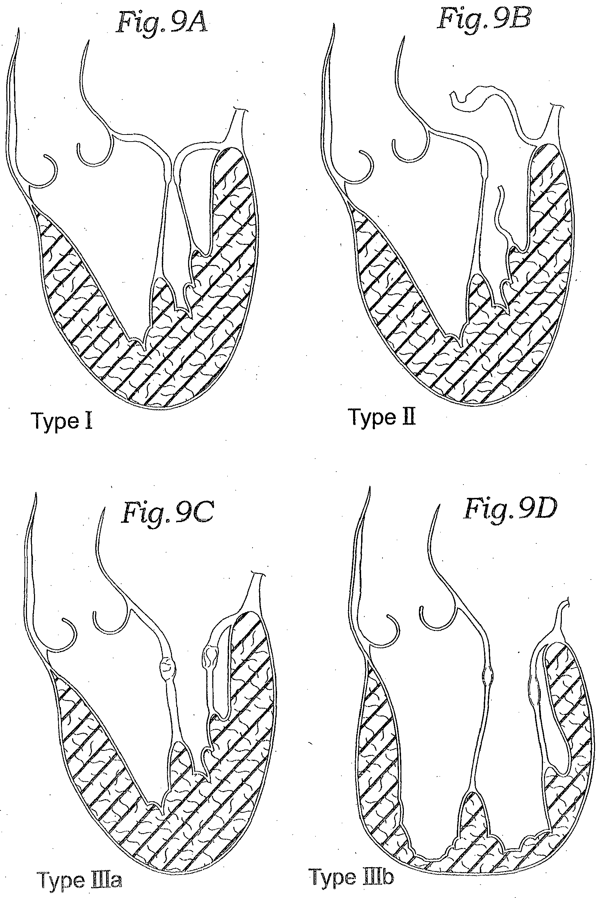

Carpentier's functional classification of the types of leaflet and chordal motion associated with mitral regurgitation may be seen with reference to Figures 9A-9D. In Type I, Figure 9A, the leaflet motion is normal. Type II (seen in Figure 9B) mitral regurgitation is due to leaflet prolapse or excessive motion. Type III (restricted leaflet motion) is subdivided into restriction during diastole Type IIIa (Figure 9C) or systole Type IIIb (Figure 9D). Type IIIb (Figure 9C) is typically seen in patients with ischemic mitral regurgitation. The course of the leaflets during the cardiac cycle is represented by the dotted lines. (Derived from Carpentier A: Cardiac valve surgery: the "French correction." J Thorac Cardiovasc Surg 86: 323, 1983.)

Various surgical techniques may be used to repair diseased or damaged mitral and tricuspid valves. These include but are not limited to annuloplasty (i.e., contracting the valve annulus to restore the proper size and shape of the valve), quadrangular resection of the leaflets (i.e., removing tissue from enlarged or misshapen leaflets), commissurotomy (i.e., cutting the valve commissures to separate the valve leaflets), shortening and transposition of the chordae tendoneae, reattachment of severed chordae tendoneae or papillary muscle tissue, and decalcification of valve and annulus tissue.

In patients with degenerative mitral valve disease, valve repairs using mitral valvuloplasty valve reconstruction, or annuloplasty have been the standards for surgical correction of mitral regurgitation and have provided good long-term results. A rigid support ring (e.g., Carpentier-Edwards Classic®), a semi-flexible ring (e.g., Carpentier-Edwards Physio®), or a flexible ring (e.g., Cosgrove-Edwards®) may be used. These rings are typically D-shaped with a minor/major axis ratio of about 3:4. Some rings are flat or planar, while others exhibit three-dimensional bows, typically along the anterior segment. Not all physicians agree which ring is appropriate for any one condition.

Despite accepted treatments for correcting mitral regurgitation, there is a need for a simpler and more effective approach that takes into account more of the common pathologies.

### Summary of the Invention

The present invention provides, in one aspect, a set of mitral annuloplasty rings according to claim 1 attached.

In particular, the set of mitral annuloplasty rings each comprises a ring body arranged around a flow axis having an upward direction and a downward direction. The downward direction corresponds to the direction of blood flow through the mitral valve annulus when the annuloplasty ring is implanted. The ring body generally D-shaped in plan view defines a minor axis extending between and bisecting the anterior segment and posterior portion and a major axis extending perpendicularly thereto, the major and minor axes being generally perpendicular to the flow axis and each having dimensions across the ring body. Each ring has a nominal orifice size, measured across the major axis in even mm increments.

The set of rings is progressively sized to take into account more of the common pathologies. More specifically, the proportional shapes of each ring as the orifice size changes are not the same. In a preferred embodiment, the larger rings have larger minor axis dimensions relative to their major axes.

### Brief Description of the Drawings

Features and advantages of the present invention will become appreciated as the same become better understood with reference to the specification, claims, and appended drawings wherein:
Figure 1A is a diagram of a normal mitral annulus, leaflets, and connected chordae and papillary muscles shown laid flat or unrolled;
Figure 1B is a "radial" sectional view through one of the papillary muscles of Figure 1 A
Figures 2-8 are diagrams from the same viewpoints as Figures 1A and 1B demonstrating various causes of pure mitral regurgitation as follows:
Figures "A-"B illustrate infective endocarditis;
Figures 3A-3B illustrate floppy mitral valve;
Figures 4A-4B illustrate floppy mitral valve with ruptured chordae;
Figures 5A-5B illustrate rheumatic heart disease;
Figures 6A-6B illustrate papillary muscle dysfunction (coronary);
Figures 7A-7B illustrate papillary muscle dysfunction (infiltrative); and
Figures 8A-8B illustrate annular calcification;
Figures 9A-9D illustrate Carpentier's functional classification of mitral regurgitation, namely: Type I: normal leaflet motion. Type II: increased leaflet motion (leaflet prolapse). Type III: restricted leaflet motion; IIIa, restriction in diastole and systole; IIIb, restriction in systole;

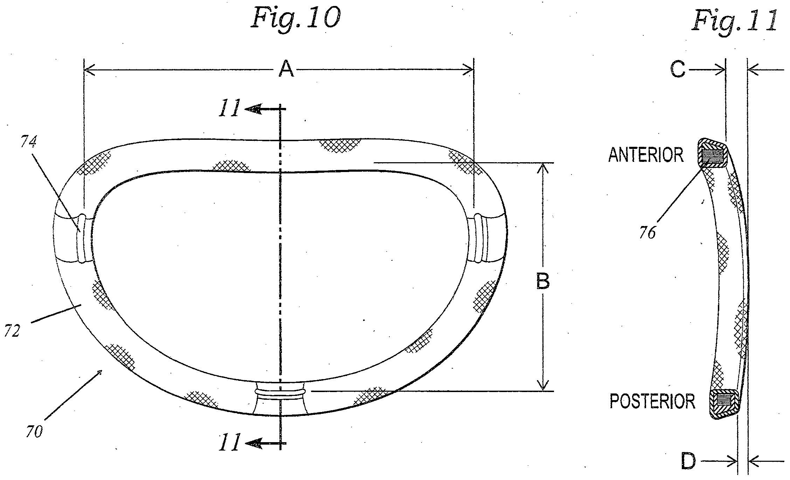

Figures 10 and 11 are plan and section views of an exemplary annuloplasty ring of the present invention;

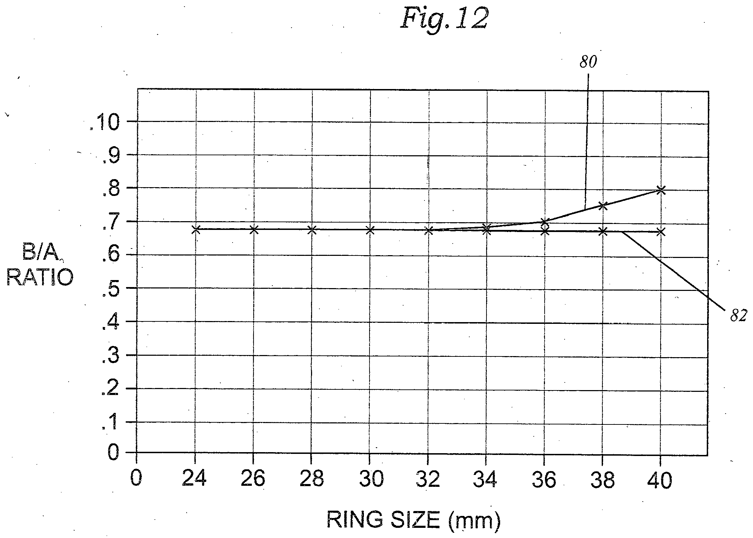

Figure 12 is a graph showing the changing minor/major axis proportion of the exemplary ring; and

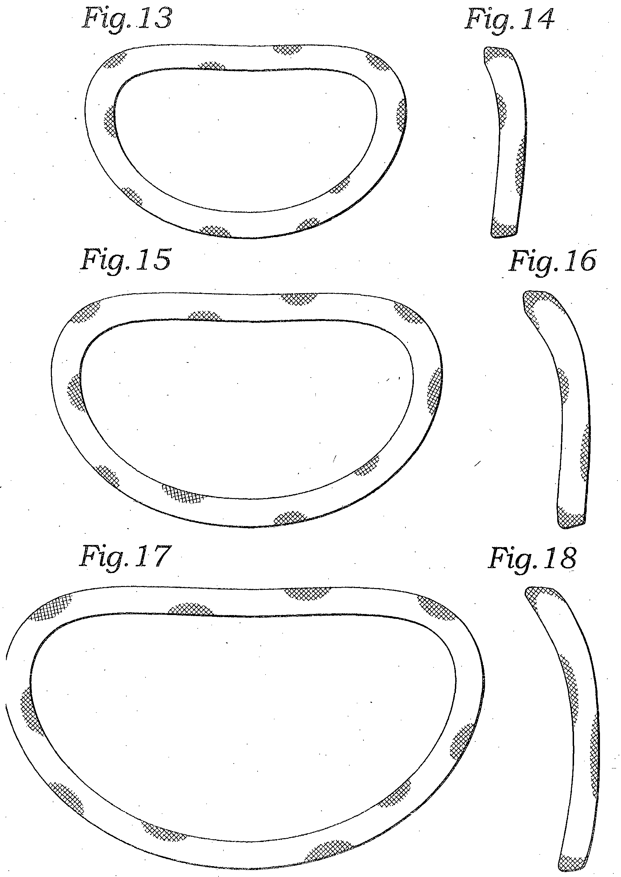

Figures 13-18 show plan and side views of several different sized rings of the present invention.

### Detailed Description of the Preferred Embodiments

The present invention provides a novel set of annuloplasty rings for correcting pathologies resulting in mitral regurgitation.

Figures 10 and 11 are plan and section views of an exemplary annuloplasty ring 70 of the present invention. The ring 70 is shown with a fabric covering 72 over a structural interior support or body 74. Typically a suture-permeable interface 76 fills the space between the covering 72 and interior body 74.

The ring 70 in the plan view of Figure 10 has a minor axis dimension B and a major axis dimension A. Figure 11 shows preferred heights above a datum plane, with the center of the anterior segment rising to height C and the center of the posterior segment rising to height D. The preferred ratio of C/D is about 3:1, with the smallest rings rising to 3 mm on the anterior side and the largest to about 6 mm.

The interior body 74 of the present invention in one embodiments is desirably made of material(s) that are "generally rigid" and will initially resist distortion when subjected to the stress imparted thereon by the mitral valve annulus of an operating human heart. In this sense, "distortion" means substantial permanent deformation from a predetermined or manufactured shape; the opposite concept of which is "elastic" meaning the ability to recover the ring shape in the absence of an external force. A number of "generally rigid" materials can be utilized that will perform this function, including various bio-compatible polymers and metals and/or alloys. Certain polyesters that resist distortion and also rapid degradation within the body may be used (a material that degrades slowly may provide the required initial support). In a preferred embodiment, at least an inner core or body of the annuloplasty ring of the present invention is made of a suitable metal, such as titanium or its alloys, or ELGILOY made by Elgiloy, L.P. of Elgin, Ill., U.S.A. The core or ring body may be one piece, or may include a plurality of concentric or otherwise cooperating elements.

The interface 76 is a molded silicone tube or band around the ring body 74 and the fabric covering on the exterior of the ring is desirably Dacron (polyethylene terephthalate). The tubular fabric covering around the silicone sleeve provide an interface for securing the annuloplasty ring to the mitral annulus, although other interfaces are contemplated. For example, rings having outward hooks or barbs are known in the art.

Typical annuloplasty support rings have a long or major dimension and a short or minor dimension, with the conventional ratio of the minor to major dimension being at most 3:4 (75%), and typically less. The present invention provides an annuloplasty ring that has a gradually increasing minor axis dimension B to major axis dimension A ratio. The dimensions A and B are measured to the inner edge of the body 74. This increasing dimensional ratio provides rings in the larger sizes that are more suited to correcting conditions where the mitral leaflet is floppy, such as the conditions shown in Figures 2-4, and in general for Type II pathologies seen in Figure 9B. Typically, larger patients exhibit this general condition leading to regurgitation as opposed to smaller patients, for which rings having more conventional B/A ratios are more appropriate.

The following table indicates the actual values of the major and minor axes as measured across the interior of the ring body 74 (dimensions A and B, respectively, in Figure 10) for nine different exemplary rings, and also gives the ratios of the minor axis to the major axis. The ring sizes are given in even 2 mm increments as measured across the major axis. Such rings will have distinct packaging so as to be labeled with the particular size.

| Ring size (mm) | Major axis (mm) | Minor Axis (mm) | B/A ratio |
|---|---|---|---|
| 24 | 24.0 | 16.5 | 0.6875 |
| 26 | 26.0 | 17.7 | 0.6808 |
| 28 | 28.0 | 18.9 | 0.6750 |
| 30 | 30.0 | 20.4 | 0.6800 |
| 32 | 32.0 | 21.9 | 0.6844 |
| 34 | 34.0 | 23.5 | 0.6912 |
| 36 | 36.0 | 25.5 | 0.7083 |
| 38 | 38.0 | 28.5 | 0.7500 |
| 40 | 40.0 | 32.0 | 0.8000 |

Figure 12 is a graph showing the changing minor/major axis proportion of the exemplary ring along line 80 as compared with a line 82 for a prior art ring, the Carpentier-Edwards Physio® ring. This shows the divergence of the ring proportions starting at around the 32 mm ring.

Figures 13-18 show plan and side views of several different sized rings of the present invention for comparison. Figures 13-14 show a 24 mm ring, Figures 15-16 show a 32 mm ring, and Figures 17-18 show a 40 mm ring. The overall "look" of the rings are the same though the B/A ration increases in the larger rings.

While the invention has been described in its preferred embodiments, it is to be understood that the words which have been used are words of description and not of limitation. Therefore, changes may be made within the appended claims without departing from the true scope of the invention.

## Claims

1. A set of mitral annuloplasty rings (70) comprising:
a set of closed and generally rigid ring bodies (74) with a fabric covering (72) over the structural interior generally rigid ring bodies (74), which are arranged around a flow axis having an upward direction and a downward direction, the downward direction corresponding to the direction of blood flow through the mitral valve annulus when the annu-loplasty ring (70) is implanted, the ring bodies (74) being generally D-shaped in plan view and defining a major axis A and a minor axis B, and each ring (70) having a nominal orifice size, measured across the major axis A in even mm increments; and
wherein the minor/major axis proportional shape of the ring bodies (74) increases for each generally rigid ring body with increasing nominal orifice sizes of the ring bodies starting at a nominal orifice size of 32mm.

## Patentansprüche

1. Satz mitraler Anuloplastieringe (70), umfassend:
einen Satz geschlossener und allgemein steifer Ring-Hauptteile (74) mit einem Stoffüberzug (72) über den strukturellen inneren allgemein steifen Ring-Hauptteilen (74), die um eine Durchflussachse mit einer Aufwärtsrichtung und einer Abwärtsrichtung angeordnet sind, wobei die Abwärtsrichtung der Richtung des Blutstroms durch den Mitralklappenanulus entspricht, wenn der Anuloplastiering (70) implantiert ist, die Ring-Hauptteile (74) allgemein D-förmig in der Draufsicht sind und eine Hauptachse A und eine Nebenachse B festlegen und jeder Ring (70) eine Nennöffnungsgröße aufweist, gemessen über die Hauptachse A in gleichen mm-Inkrementen; und
wobei sich die proportionale Form über Neben-/Hauptachse der Ring-Hauptteile (74) allgemein für jeden Ring-Hauptteil mit steigenden Nennöffnungsgrößen der Ring-Hauptteile vergrößert, beginnend bei einer Nennöffnungsgröße von 32 mm.

## Revendications

1. Jeu d'anneaux (70) d'annuloplastie mitrale, comprenant :
un jeu de corps (74) annulaires fermés et d'une manière générale rigides avec un recouvrement (72) de tissu sur les corps (74) annulaires intérieurs de structure, d'une manière générale rigides, qui sont disposés autour d'un axe d'écoulement ayant un sens vers le haut et un sens vers le bas, le sens vers le haut correspondant au sens du flux sanguin dans l'anneau de la valvule mitrale, lorsque l'anneau (70) d'annuloplastie est implanté, les corps (74) annulaires étant, d'une manière générale, en forme de D dans une vue en plan et définissant un grand axe A et un petit axe B, et chaque anneau (70) ayant une dimension nominale d'orifice mesurée suivant le grand axe A en incréments égaux en millimètres et
dans lequel la forme proportionnelle petit axe/grand axe des corps (74) annulaires augmente pour chaque corps annulaire, d'une manière générale, rigide au fur et à mesure qu'augmentent les dimensions nominales de l'orifice des corps annulaires en partant d'une dimension nominale d'orifice de 32 mm.
